# EUROPEAN PATENT APPLICATION

(11) **EP 4 624 445 A1**
(43) Date of publication of application: **01.10.2025**
(21) Application number: 22966199.6
(22) Date of filing: 24.11.2022
(51) Int. Cl.: C07C 15/08, C10G 69/04, C07C 4/06, C10G 11/18

(54) **FLUIDIZED BED DEVICE AND METHOD FOR PREPARING AROMATIC HYDROCARBONS FROM NAPHTHA**

(71) Applicant: Dalian Institute Of Chemical Physics, Chinese Academy Of Sciences, Liaoning 116023 (CN); China Shenhua Coal to Liquid and Chemical Co., Ltd, Beijing 100011 (CN)
(72) Inventor: ZHANG, Tao, Dalian, Liaoning 116023 (CN); YE, Mao, Dalian, Liaoning 116023 (CN); YAN, Guochun, Beijing 100011 (CN); LIU, Zhongmin, Dalian, Liaoning 116023 (CN); ZHANG, Jinling, Dalian, Liaoning 116023 (CN); ZHANG, Jiming, Beijing 100011 (CN); JIA, Jinming, Dalian, Liaoning 116023 (CN); WEN, Liang, Beijing 100011 (CN); MA, Xiangang, Dalian, Liaoning 116023 (CN); LIN, Huadong, Beijing 100011 (CN); TANG, Hailong, Dalian, Liaoning 116023 (CN); MA, Zhichao, Beijing 100011 (CN); ZHANG, Cheng, Dalian, Liaoning 116023 (CN); WANG, Xiangao, Dalian, Liaoning 116023 (CN); WANG, Jing, Dalian, Liaoning 116023 (CN); GAO, Mingbin, Dalian, Liaoning 116023 (CN); LI, Xue, Dalian, Liaoning 116023 (CN); XU, Shuliang, Dalian, Liaoning 116023 (CN); LI, Hua, Dalian, Liaoning 116023 (CN); LI, Chenggong, Dalian, Liaoning 116023 (CN)
(74) Representative: Bayramoglu et al.
(86) International application number: PCT/CN2022/134159
(87) International publication number: WO 2024/108508

(57) **Abstract**

The present application discloses a naphtha-to-aromatics fluidized bed device, the device at least comprises: a light hydrocarbon aromatization reactor; and a naphtha-to-aromatics reactor; the high-temperature regenerated catalyst is first directed into the light hydrocarbon aromatization reactor, and after cooling, subsequently introduced into the naphtha-to-aromatics reactor. The method of the present application comprises using the above-mentioned device and a metal molecular sieve bifunctional catalyst. Under the action of the catalyst in the naphtha to aromatics reactor, naphtha is converted into a product gas containing aromatics, light alkanes, and other components. Light alkanes and the like separated from the product gas are further converted into aromatics and other components in the light hydrocarbon aromatization reactor. The method of the present application enables efficient and highly selective conversion of linear and branched aliphatic hydrocarbons into aromatics, with para-xylene content in the xylene mixture exceeding >50 wt%.

## Description

### TECHNICAL FIELD

The present application relates to a fluidized bed device and a method for use thereof, and specifically relates to a fluidized bed device for producing aromatics from naphtha and a method thereof, belonging to the technical field of chemical engineering.

### BACKGROUND

Aromatics (benzene, toluene, and xylene, collectively referred to as BTX) are important organic chemical raw materials. Among them, para-xylene (PX) is the most noteworthy product in aromatics, mainly used to produce terephthalic acid (PTA), polyethylene terephthalate (PET), polybutylene terephthalate (PBT), and polytrimethylene terephthalate (PTT). In recent years, China's production and consumption of PX have continued to grow. In 2021, China's total PX imports amounted to approximately 13.65 million tons, with an external dependency of about 38%.

Naphtha catalytic reforming technology is the primary technical route for producing aromatics. The composition of naphtha is highly complex, as it serves not only as the main feedstock for catalytic reforming but also as a key raw material for ethylene production via cracking. The composition of naphtha plays a decisive role in the economic efficiency of the device. Generally, feedstock with high aromatic potential content and a suitable distillation range is favorable for catalytic reforming, whereas feedstock with high linear and branched aliphatic hydrocarbon content and low naphthene and aromatic content is suitable for ethylene cracking. To fully utilize naphtha resources and improve economic efficiency, it is necessary to separate linear and branched aliphatic hydrocarbons from naphthenes and aromatics in naphtha, with the former used as feedstock for ethylene production and the latter as feedstock for catalytic reforming devices.

Naphtha fractions have a wide distillation range, making it difficult for conventional separation methods to efficiently separate linear and branched aliphatic hydrocarbons from naphthenes and aromatics. Additionally, catalytic reforming technology struggles to convert linear and branched aliphatic hydrocarbons into aromatics. Naphtha feedstock for catalytic reforming typically requires distillation to remove light fractions (boiling below 60°C), thereby improving the aromatic potential content of the catalytic reforming feedstock. However, fractions with boiling points above 60°C still contain significant amounts of linear and branched aliphatic hydrocarbons that are difficult to convert into aromatics. Therefore, the high-selectivity conversion of linear and branched aliphatic hydrocarbons into aromatics has been a key focus and challenge in the development of naphtha-to-aromatics technology.

Due to thermodynamic equilibrium limitations, para-xylene accounts for only ~24% of the xylene mixture produced by naphtha catalytic reforming devices, necessitating further para-xylene production through isomerization-separation processes. Thus, increasing the para-xylene content in the xylene mixture is an important approach to reducing energy consumption in para-xylene production.

### SUMMARY

According to one aspect of the present application, a fluidized bed device is provided that can produce aromatics from naphtha feedstock with low aromatic potential content. This device increases the para-xylene content in mixed xylenes and reduces production energy consumption.

The naphtha components described in the present application include C₄-C₁₂ linear aliphatic hydrocarbons, branched aliphatic hydrocarbons, cycloalkanes, and aromatics.

The aromatics described in the present application refer to benzene, toluene, and xylene, collectively referred to as BTX.

The naphtha-to-aromatics fluidized bed device comprises: a light hydrocarbon aromatization reactor; and a naphtha-to-aromatics reactor; wherein
the light hydrocarbon aromatization reactor includes at least one inlet for introducing a raw material and a high-temperature catalyst; and at least one outlet of the light hydrocarbon aromatization reactor is connected to the naphtha-to-aromatics reactor for delivering a catalyst and a generated light hydrocarbon aromatization product gas to the naphtha-to-aromatics reactor;
the naphtha-to-aromatics reactor is configured to introduce naphtha to react with a catalyst from the light hydrocarbon aromatization reactor to produce a product gas flow containing BTX. Among them, the product gas flow containing BTX includes light hydrocarbon aromatization product gas.

When entering the naphtha-to-aromatics reactor, the catalyst temperature decreases. At this point, upon contact with naphtha, it helps eliminate localized high-temperature zones in the naphtha-to-aromatics reactor, thereby effectively reducing light alkane yield and increasing aromatic yield.

Preferably, the naphtha-to-aromatics reactor is further provided with a product gas delivery pipe I for outputting the product gas flow containing BTX to a downstream section.

Preferably, the device further comprises a regenerator, and at least one inlet of the light hydrocarbon aromatization reactor is connected to the regenerator for obtaining a high-temperature regenerated catalyst generated by the regenerator.

Preferably, the light hydrocarbon aromatization reactor is divided from top to bottom into at least a gas-solid separation zone II and a light hydrocarbon aromatization reaction zone which are connected to form a bed reactor; the gas-solid separation zone II is provided with a gas-solid separation unit II and a gas collection chamber II, the gas outlet of the gas-solid separation unit II is connected to the gas collection chamber II, and a bed reactor distributor is provided in the inner lower part of the light hydrocarbon aromatization reaction zone for feeding a bed reactor feedstock.

Preferably, the gas collection chamber II is located at the inner top of the bed reactor.

Furthermore, the bed reactor feedstock comprises C₄ and C₅ hydrocarbons.

Preferably, the bed reactor feedstock comprises C₃, C₄ and C₅ hydrocarbons.

Preferably, the light hydrocarbon aromatization reactor further comprises a riser reactor in addition to the bed reactor, wherein the inlet end of the riser reactor is connect to the regenerator; the outlet end of the riser reactor extends into the lower section of the light hydrocarbon aromatization reaction zone, with the catalyst outlet of the gas-solid separation unit II located above it.

Preferably, the inlet end of the riser reactor is further configured to introduce a catalyst and a riser reactor feedstock.

Preferably, the naphtha-to-aromatics reactor is divided from top to bottom into at least a gas-solid separation zone I and a naphtha-to-aromatics reaction zone which are connected; the gas-solid separation zone I is provided with a gas-solid separation unit I and a gas collection chamber I; the gas outlet of the gas-solid separation unit I is connect to the gas collection chamber I; the lower section of the naphtha-to-aromatics reaction zone is provided with a naphtha-to-aromatics reactor distributor for feeding naphtha feedstock.

Preferably, the gas-solid separation unit I employs at least one set of gas-solid cyclone separators, each set comprising a first-stage gas-solid cyclone separator and a second-stage gas-solid cyclone separator.

Preferably, the gas collection chamber I is located at the inner top of the naphtha-to-aromatics reactor.

Preferably, the gas collection chamber I is connected to the product gas delivery pipe I.

Preferably, the gas-solid separation zone I is connected to the gas-solid separation zone II, and the naphtha-to-aromatics reaction zone of the naphtha-to-aromatics reactor is connected to the light hydrocarbon aromatization reaction zone.

Preferably, the gas-solid separation zone I is connected to the gas collection chamber II through a product gas delivery pipe II.

Preferably, a light hydrocarbon aromatization slide valve is provided on the pipeline connecting the naphtha-to-aromatics reaction zone of the naphtha-to-aromatics reactor and the light hydrocarbon aromatization reaction zone.

Preferably, the outlet of the light hydrocarbon aromatization reaction zone is positioned higher than the inlet of the naphtha-to-aromatics reaction zone of the naphtha-to-aromatics reactor.

Preferably, the regenerator is divided from top to bottom into at least a gas-solid separation zone III and a catalyst regeneration zone which are connected; the gas-solid separation zone III is provided with a regenerator gas-solid separation unit and a regenerator gas collection chamber; the gas outlet of the regenerator gas-solid separation unit is connected to the regenerator gas collection chamber; the regenerator gas collection chamber is provided with a flue gas delivery pipe; the lower section of the catalyst regeneration zone is provided with a regenerator distributor for introducing regeneration gas.

Preferably, the catalyst regeneration zone is sequentially connected to the light hydrocarbon aromatization reactor through a regenerator stripper and a regenerated catalyst slide valve; the inlet pipe of the regenerator stripper extends into the regenerator shell, located above the regenerator distributor, and the catalyst outlet end of the regenerator gas-solid separation unit is located above the open end of the inlet pipe of the regenerator stripper.

Preferably, the regenerator gas collection chamber is located at the inner top of the regenerator shell.

Preferably, at least one outlet of the naphtha-to-aromatics reactor is further connected to the inlet of the regenerator for delivering a spent catalyst generated in the naphtha-to-aromatics reactor to the regenerator, wherein the regenerator is configured to introduce a regeneration gas and convert the spent catalyst into a regenerated catalyst.

Preferably, the naphtha-to-aromatics reaction zone of the naphtha-to-aromatics reactor is sequentially connected to the inlet of the regenerator through a reactor stripper, a spent catalyst slide valve and a spent catalyst delivery pipe; the inlet pipe of the reactor stripper extends into the naphtha-to-aromatics reactor shell, located above the naphtha-to-aromatics reactor distributor, and the catalyst outlet end of the gas-solid separation unit I is located above the open end of the inlet pipe of the reactor stripper.

Preferably, the catalyst inlet of the regenerator is provided on the regenerator shell.

According to another aspect of the present application, a method for producing aromatics from naphtha is provided. The method comprises: preparing aromatics using the aforementioned naphtha-to-aromatics fluidized bed device.

Further, the method comprises:
introducing the raw material and the high-temperature catalyst into the light hydrocarbon aromatization reactor to generate the light hydrocarbon aromatization product gas;
introducing naphtha and the catalyst and the light hydrocarbon aromatization product gas from the light hydrocarbon aromatization reactor into the naphtha-to-aromatics reactor to generate a product gas flow containing BTX.

Preferably, the catalyst is a metal molecular sieve bifunctional catalyst.

Preferably, the metal molecular sieve bifunctional catalyst employs a metal-modified HZSM-5 zeolite molecular sieve;
a metal used for a metal modification is at least one selected from the group consisting of La, Zn, Ga, Fe, Mo, and Cr;
the metal modification comprises: placing an HZSM-5 zeolite molecular sieve in a metal salt solution, and carrying out an impregnation, a drying and a calcination to obtain the metal-modified HZSM-5 zeolite molecular sieve.

Preferably, the components of the light hydrocarbon aromatization product gas comprise: BTX, light olefins and H₂.

Preferably, in addition to BTX, the product gas flow containing BTX further comprises: light olefins, hydrogen, light alkanes, combustible gas, heavy aromatics and unconverted naphtha.

Preferably, the light olefins refer to ethylene and propylene.

Preferably, the light alkanes refer to ethane and propane;
the combustible gas comprises methane and CO;
the heavy aromatics refer to aromatic hydrocarbons with nine or more carbon atoms per molecule.

Preferably, introducing the raw material and the high-temperature catalyst into the light hydrocarbon aromatization reactor to generate light hydrocarbon aromatization product gas specifically comprises:
feeding the bed reactor feedstock into the light hydrocarbon aromatization reaction zone through the bed reactor distributor, contacting with the catalyst introduced into the bed reactor to generate light hydrocarbon aromatization product gas.

Preferably, the bed reactor feedstock comprises C₄ and C₅ hydrocarbons.

Preferably, the bed reactor feedstock comprises C₃, C₄ and C₅ hydrocarbons.

Preferably, the C₄ and C₅ hydrocarbons are derived from C₄ and C₅ hydrocarbons separated from the product gas flow.

Preferably, the C₃, C₄ and C₅ hydrocarbons are derived from C₃, C₄ and C₅ hydrocarbons separated from the product gas flow.

Preferably, the process conditions for the light hydrocarbon aromatization reaction zone are: gas superficial velocity of 0.5-2.0 m/s, reaction temperature of 550-665°C, reaction pressure of 100-500 kPa, bed density of 150-700 kg/m³.

Optionally, the gas superficial velocity is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the reaction temperature is independently selected from any value among 550°C, 560°C, 570°C, 580°C, 590°C, 600°C, 610°C, 620°C, 630°C, 640°C, 650°C, 660°C, 665°C or any range between two values.

Optionally, the reaction pressure is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among 150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

Preferably, the method further comprises: removing the catalyst entrained in the light hydrocarbon aromatization product gas in the gas-solid separation zone II of the light hydrocarbon aromatization reactor.

Preferably, the catalyst in the light hydrocarbon aromatization reaction zone enters the naphtha-to-aromatics reactor through the light hydrocarbon aromatization slide valve.

Preferably, removing catalyst entrained in the light hydrocarbon aromatization product gas in the gas-solid separation zone II specifically comprises:
the light hydrocarbon aromatization product gas enters the gas-solid separation unit II to remove the catalyst entrained in it, then enters the gas collection chamber II, and is delivered to the gas-solid separation zone I of the naphtha-to-aromatics reactor through the product gas delivery pipe II.

Preferably, introducing naphtha into the naphtha-to-aromatics reaction zone of the naphtha-to-aromatics reactor through the naphtha-to-aromatics reactor distributor, contacting with the catalyst from the light hydrocarbon aromatization reactor to generate a product gas flow containing BTX, while the catalyst becomes coked and converts into the spent catalyst.

Preferably, the method further comprises: removing the spent catalyst entrained in the product gas flow containing BTX in the gas-solid separation zone I of the naphtha-to-aromatics reactor and delivering to downstream section.

Preferably, the naphtha is at least one selected from the group consisting of coal direct liquefaction naphtha, coal indirect liquefaction naphtha, straight-run naphtha and hydrocracked naphtha.

Preferably, the naphtha further comprises unconverted naphtha separated from the product gas flow, wherein the main components of the unconverted naphtha are linear aliphatic hydrocarbons, branched aliphatic hydrocarbons and naphthenes of C₄-C₁₂.

Preferably, the process conditions for the naphtha-to-aromatics reaction zone are: gas superficial velocity of 0.5-2.0 m/s, reaction temperature of 500-650°C, reaction pressure of 100-500 kPa, bed density of 150-700 kg/m³.

Optionally, the gas superficial velocity is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the reaction temperature is independently selected from any value among 500°C, 520°C, 530°C, 540°C, 550°C, 560°C, 570°C, 580°C, 590°C, 600°C, 610°C, 620°C, 630°C, 640°C, 650°C or any range between two values.

Optionally, the reaction pressure is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among 150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

Preferably, the method further comprises: introducing the regeneration gas and the spent catalyst into the regenerator to obtain the high-temperature regenerated catalyst, which is delivered to the light hydrocarbon aromatization reactor.

Preferably, the regeneration gas is introduced into the regeneration zone of the regenerator through the regenerator distributor.

Preferably, the regeneration gas is at least one selected from the group consisting of oxygen, air and oxygen-enriched air.

Preferably, the carbon content in the spent catalyst is in a range from 1.0 wt% to 3.0 wt%.

Preferably, the carbon content in the regenerated catalyst is ≤0.5 wt%.

Preferably, the process conditions for the regeneration zone of the regenerator are: gas superficial velocity of 0.5-2.0 m/s, regeneration temperature of 600-750°C, regeneration pressure of 100-500 kPa, bed density of 150-700 kg/m³.

Optionally, the gas superficial velocity is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the regeneration temperature is independently selected from any value among 600°C, 615°C, 630°C, 645°C, 670°C, 685°C, 700°C, 715°C, 730°C, 745°C, 750°C or any range between two values.

Optionally, the regeneration pressure is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

Preferably, the coke on the spent catalyst reacts with the regeneration gas to generate a flue gas; the flue gas enters the gas-solid separation zone III to remove the regenerated catalyst entrained in it.

Preferably, the flue gas entering the gas-solid separation zone III to remove the regenerated catalyst entrained in it specifically comprises: the flue gas first enters the regenerator gas-solid separation unit to remove the regenerated catalyst entrained in it, then flows through the regenerator gas collection chamber and flue gas delivery pipe to enter downstream section.

Preferably, the regenerated catalyst enters the light hydrocarbon aromatization reactor through the regenerator stripper and the regenerated catalyst slide valve.

Preferably, the method further comprises: feeding the riser reactor feedstock into the inlet end of the riser reactor of the light hydrocarbon aromatization reactor; the regenerated catalyst flows through the regenerator stripper and regenerated catalyst slide valve into the riser reactor, where the riser reactor feedstock is converted into a BTX-containing flow under the action of the regenerated catalyst, which then enters the lower section of the light hydrocarbon aromatization reaction zone in the bed reactor through the outlet end of the riser reactor.

Preferably, the method further comprises: introducing the catalyst into the inlet end of the riser reactor of the light hydrocarbon aromatization reactor, which enters the bed reactor through the riser reactor.

Preferably, the riser reactor feedstock comprises water vapor and light alkanes separated from the product gas flow. Since light alkanes are highly stable and require elevated reaction temperatures, the present application introduces a process where the high-temperature regenerated catalyst is first fed into the light hydrocarbon aromatization reactor to contact light alkanes, C₄ and C₅ hydrocarbons. This enables the aromatization reaction of these hydrocarbons under catalytic action, thereby enhancing aromatic yield.

Preferably, the water vapor content in the riser reactor feedstock is in a range from 0 wt% to 80 wt%.

Preferably, the process conditions for the riser reactor are: gas superficial velocity of 3.0-10.0 m/s, temperature of 580-700°C, pressure of 100-500 kPa, bed density of 50-150 kg/m³.

Optionally, the gas superficial velocity is independently selected from any value among 3.0m/s, 3.5m/s, 4.0m/s, 4.5m/s, 5.0m/s, 5.5m/s, 6.0m/s, 6.5m/s, 7.0m/s, 7.5m/s, 8.0m/s, 8.5m/s, 9.0m/s, 9.5m/s, 10.0m/s or any range between two values.

Optionally, the temperature is independently selected from any value among 580°C, 590°C, 600°C, 610°C, 620°C, 630°C, 640°C, 650°C, 660°C, 670°C, 680°C, 690°C, 700°C or any range between two values.

Optionally, the pressure is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among 50kg/m³, 60kg/m³, 70kg/m³, 80kg/m³, 90kg/m³, 100kg/m³, 110kg/m³, 120kg/m³, 130kg/m³, 140kg/m³, 150kg/m³ or any range between two values.

Preferably, the components of the BTX-containing flow comprise: BTX, light olefins and H₂.

Preferably, the method further comprises:
the spent catalyst in the naphtha-to-aromatics reaction zone of the naphtha-to-aromatics reactor is introduced into the reactor stripper, stripped, and then flows through the spent catalyst slide valve and the spent catalyst delivery pipe into a downstream section.

Preferably, the downstream section is the regenerator.

In the present application, the naphtha feedstock has an aromatic potential content of 0-80 wt%, with a single-pass conversion rate of 70-95 wt%. Using the naphtha-to-aromatics fluidized bed device and the corresponding method described herein, the final product distribution is as follows: 60-75 wt% BTX, 6-14 wt% light olefins, 3-7 wt% hydrogen, 3-8 wt% light alkanes, 4-6 wt% combustible gas, 4-8 wt% heavy aromatics, and 0.5-1 wt% coke. The para-xylene content in the mixed xylenes is 50-65 wt%.

The beneficial effects of the present application include:
1) The present invention can efficiently and selectively convert linear and branched aliphatic hydrocarbons into aromatics, and has a wide range of raw material adaptability, and can use naphtha with low aromatic potential content as a raw material to prepare aromatics.
2) The present application realizes the aromatization of light alkanes, C₄, and Cs hydrocarbons through the light hydrocarbon aromatization reactor and a metal molecular sieve bifunctional catalyst, greatly improving the aromatics yield of naphtha-to-aromatics technology.
3) The aromatic product produced by the present application has a p-xylene content in the xylene mixture of >50wt%, which is much higher than the thermodynamic equilibrium content (about 24wt%), which can effectively increase the p-xylene yield and significantly reduce the energy consumption for separating pxylene.
4) In the present application, unconverted naphtha is separated from the product gas and recycled to the naphtha-to-aromatics reactor, part of the light alkanes are separated from the product gas and recycled to the riser reactor in the light hydrocarbon aromatization reactor, C₃, C₄ and C₅ hydrocarbons are separated from the product gas and recycled to the bed reactor in the light hydrocarbon aromatization reactor, and a regenerator is provided to regenerate the catalyst, thereby realizing the closed-loop preparation of aromatics.
5) The present application connects the high-temperature light hydrocarbon aromatization reactor and the relatively low-temperature naphtha-to-aromatics reactor in series. The high-temperature catalyst first enters the light hydrocarbon aromatization reactor, and the light alkanes, C₄, and C₅ hydrocarbons in the reactor undergo aromatization reaction under the action of the catalyst, thereby increasing the yield of aromatics; the catalyst with a lowered temperature then enters the naphtha-to-aromatics reactor and contacts with the naphtha, eliminating the local high-temperature zone in the naphtha-based aromatics reactor, thereby achieving the beneficial effect of effectively reducing the yield of light alkanes and increasing the yield of aromatics.

### BRIEF DESCRIPTION OF THE DRAWINGS

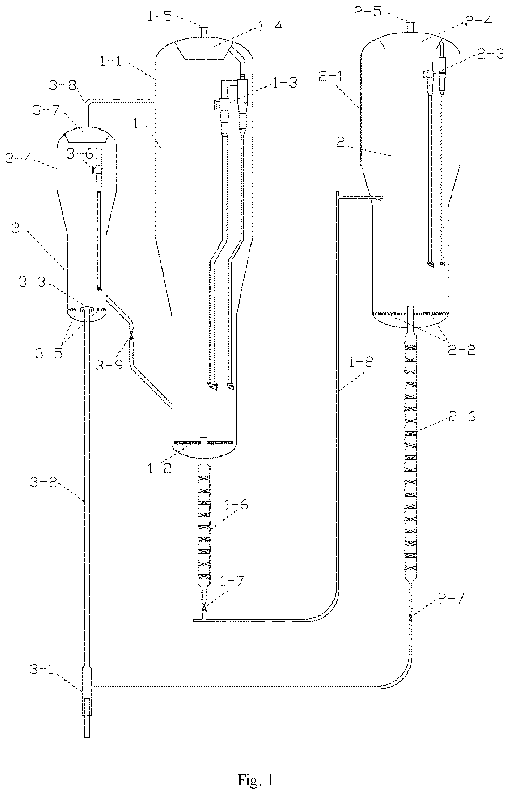

Fig. 1 is a schematic diagram of a naphtha-to-aromatics fluidized bed device according to one embodiment of the present application.

### List of Components and Reference Numerals:

1: naphtha-to-aromatics reactor; 1-1: naphtha-to-aromatics reactor shell; 1-2: naphtha-to-aromatics reactor distributor; 1-3: gas-solid separation unit I; 1-4: gas collection chamber I; 1-5: product gas delivery pipe I; 1-6: reactor stripper; 1-7: spent catalyst slide valve; 1-8: spent catalyst delivery pipe;
2: regenerator; 2-1: regenerator shell; 2-2: regenerator distributor; 2-3: regenerator gas-solid separation unit; 2-4: regenerator gas collection chamber; 2-5: flue gas delivery pipe; 2-6: regenerator stripper; 2-7: regenerated catalyst slide valve;
3: light hydrocarbon aromatization reactor; 3-1: inlet end of the riser reactor; 3-2: middle section of the riser reactor; 3-3: outlet end of the riser reactor; 3-4: bed reactor shell; 3-5: bed reactor distributor; 3-6: gas-solid separation unit II; 3-7: gas collection chamber II; 3-8: product gas delivery pipe II; 3-9: light hydrocarbon aromatization slide valve.

### DETAILED DESCRIPTION OF THE EMBODIMENTS

The present application will be described in detail below with reference to examples, but the present application is not limited to these examples.

The present application provides naphtha-to-aromatics fluidized bed device, comprising a light hydrocarbon aromatization reactor; and a naphtha-to-aromatics reactor; wherein
the light hydrocarbon aromatization reactor includes at least one inlet for introducing a raw material and a high-temperature catalyst; and at least one outlet of the light hydrocarbon aromatization reactor is connected to the naphtha-to-aromatics reactor for delivering a catalyst and a generated light hydrocarbon aromatization product gas to the naphtha-to-aromatics reactor;
the naphtha-to-aromatics reactor is configured to introduce naphtha to react with a catalyst from the light hydrocarbon aromatization reactor to produce a product gas flow containing BTX.

In the present application, the components of the naphtha include C₄-C₁₂ linear aliphatic hydrocarbons, branched aliphatic hydrocarbons, naphthenes, and aromatics.

In the present application, the term "aromatics" refers to benzene, toluene, and xylene, collectively termed BTX.

In a preferred embodiment, the naphtha-to-aromatics reactor is further provided with a product gas delivery pipe I for outputting the product gas flow containing BTX to a downstream section.

In a preferred embodiment, the device further comprises a regenerator, and at least one inlet of the light hydrocarbon aromatization reactor is connected to the regenerator for obtaining a high-temperature regenerated catalyst generated by the regenerator.

Please refer to Fig. 1, which shows a preferred embodiment of naphtha-to-aromatics fluidized bed device in the present application. The device comprises a naphtha-to-aromatics reactor 1, a regenerator 2, and a light hydrocarbon aromatization reactor 3.

The naphtha-to-aromatics reactor 1 includes: a naphtha-to-aromatics reactor shell 1-1, a naphtha-to-aromatics reactor distributor 1-2, a gas-solid separation unit I 1-3, a gas collection chamber I 1-4, a product gas delivery pipe I 1-5, a reactor stripper 1-6; a spent catalyst slide valve 1-7, a spent catalyst delivery pipe 1-8.

The naphtha-to-aromatics reactor shell 1-1 comprises a reactor upper shell and a reactor lower shell. The reactor upper shell encloses a gas-solid separation zone and the reactor lower shell encloses a naphtha-to-aromatics reaction zone; The naphtha-to-aromatics reactor shell 1-1 is equipped with the outlet of the light hydrocarbon aromatization reactor 3.

The naphtha-to-aromatics reaction zone is equipped with the distributor 1-2 at its lower section for introducing naphtha feedstock.

The naphtha-to-aromatics reactor shell 1-1 houses the gas-solid separation unit I 1-3 and gas collection chamber I 1-4; The chamber I 1-4, located at the naphtha-to-aromatics reactor shell's inner top, connects to the gas outlet of the gas-solid separation unit I 1-3 and the product gas delivery pipe I 1-5. The catalyst outlet of separation unit I 1-3 is positioned above the open end of the reactor stripper 1-6 inlet pipe.

The reactor stripper 1-6 is located below the naphtha-to-aromatics reaction zone; the inlet of the reactor stripper 1-6 is located inside the naphtha-to-aromatics reactor shell 1-1; and the outlet of the reactor stripper 1-6 located outside the naphtha-to-aromatics reactor shell 1-1 and is connected to the spent catalyst slide valve I 1-7; the open end of the inlet of the reactor stripper 1-6 is located above the naphtha-to-aromatics reactor distributor 1-2.

The spent catalyst slide valve 1-7 is provided below the reactor stripper 1-6; the inlet of the spent catalyst slide valve 1-7 is connected to the outlet of the reactor stripper 1-6, the outlet of the spent catalyst slide valve 1-7 is connected to the inlet of the spent catalyst delivery pipe 1-8, and the outlet of the spent catalyst delivery pipe 1-8 is connected to the regenerator shell 2-1.

The spent catalyst slide valve 1-7 is used to control the circulation amount of the spent catalyst.

In a preferred embodiment, the gas-solid separation unit I 1-3 uses one or more groups of gas-solid cyclone separators, and each group of gas-solid cyclone separators includes a first-stage gas-solid cyclone separator and a second-stage gas-solid cyclone separator.

The regenerator 2 comprises: the regenerator shell 2-1, the regenerator distributor 2-2, the regenerator gas-solid separation unit 2-3, the regenerator gas collection chamber 2-4, the flue gas delivery pipe 2-5, the regenerator stripper 2-6, the regenerated catalyst slide valve 2-7.

The regenerator shell 2-1 comprises the regenerator upper shell and the regenerator lower shell, wherein the regenerator upper shell encloses the gas-solid separation zone, and the regenerator lower shell encloses the regeneration zone; the regenerator shell 2-1 is provided with an outlet of the spent catalyst delivery pipe 1-8.

The regenerator distributor 2-2 is provided at the lower part of the regeneration zone, and the regenerator distributor 2-2 is used for introducing the regeneration gas.

The regenerator shell 2-1 is also provided with a regenerator gas-solid separation unit 2-3 and a regenerator gas collection chamber 2-4; the regenerator gas collection chamber 2-4 is located at the inner top of the regenerator shell 2-1; the gas outlet of the regenerator gas-solid separation uint 2-3 is connected to the regenerator gas collection chamber 2-4; the regenerator gas collection chamber 2-4 is connected to the flue gas delivery pipe 2-5; the catalyst outlet end of the regenerator gas-solid separation unit 2-3 is located above the opening end of the inlet pipe of the regenerator stripper 2-6.

The regenerator stripper 2-6 is provided below the regeneration zone; the inlet of the regenerator stripper 2-6 is located inside the regenerator shell 2-1; the outlet of the regenerator stripper 2-6 is located outside the regenerator shell 2-1, and is connected to the regenerated catalyst slide valve 2-7; the opening end of the inlet of the regenerator stripper 2-6 is located above the regenerator distributor 2-2.

The regenerator stripper 2-6 is equipped with the regenerated catalyst slide valve 2-7 beneath it, the outlet of the regenerator stripper 2-6 is connected to the inlet of the regenerated catalyst slide valve 2-7.

The regenerated catalyst slide valve 2-7 is used to control the circulation amount of the regenerated catalyst.

In a preferred embodiment, the regenerator gas-solid separation unit 2-3 uses one or more groups of gas-solid cyclone separators, and each group of gas-solid cyclone separators includes the first-stage gas-solid cyclone separator and the second-stage gas-solid cyclone separator.

The light hydrocarbon aromatization reactor comprises: the inlet end of the riser reactor 3-1, the middle part of the riser reactor 3-2, the outlet end of the riser reactor 3-3, the bed reactor shell 3-4, the bed reactor distributor 3-5, the gas-solid separation unit II 3-6, the gas collection chamber II 3-7, the product gas delivery pipe II 3-8, the light hydrocarbon aromatization slide valve II 3-9.

The bed reactor shell 3-4 comprises an upper shell of the bed reactor and a lower shell of the bed reactor, the upper shell of the bed reactor encloses a gas-solid separation zone, and the lower shell of the bed reactor encloses a light hydrocarbon aromatization reaction zone; the bed reactor distributor 3-5 is provided at the inner lower part of the light hydrocarbon aromatization reaction zone; the light hydrocarbon aromatization slide valve 3-9 is provided at the outside of the light hydrocarbon aromatization reaction zone; the upper section of the riser reactor penetrates the bottom of the bed reactor and is axially inserted in the bed reactor; the outlet end of the riser reactor 3-3 is located at the inner lower part of the light hydrocarbon aromatization reaction zone.

The light hydrocarbon aromatization slide valve 3-9 is configured to deliver the catalyst to the next-stage reactor, such as the naphtha-to-aromatics reactor 1. Furthermore, the outlet of the light hydrocarbon aromatization reaction zone is positioned at a higher elevation than the inlet of the naphtha-to-aromatics reaction zone in the naphtha-to-aromatics reactor.

The gas-solid separation zone of the bed reactor is provided with the gas-solid separation unit II 3-6 and a gas collection chamber II 3-7; the gas outlet of the gas-solid separation unit II 3-6 is connected to the gas collection chamber II 3-7; the catalyst outlet of the gas-solid separation unit II 3-6 is located in the light hydrocarbon aromatization reaction zone; the gas collection chamber II 3-7 is connected to the product gas delivery pipe II 3-8 located outside the bed reactor.

In a preferred embodiment, the gas-solid separation unit II 3-6 is a gas-solid cyclone separator.

In a preferred embodiment, the gas collection chamber II 3-7 is provided at the inner top of the bed reactor; the catalyst outlet of the gas-solid cyclone separation unit 3-7 of the bed reactor is located above the outlet end of the riser reactor 3-3.

In a preferred embodiment, the bed reactor distributor 3-5 is used to introduce the bed reactor feedstock.

In a preferred embodiment, the inlet end of the riser reactor 3-1 is used to introduce the catalyst and the riser reactor feedstock.

The inlet of the light hydrocarbon aromatization reactor 3 is connected to the regenerator 2) and its outlet is connected to the naphtha-to-aromatics reactor 1.

In a preferred embodiment, The inlet end of the riser reactor 3-1 is connected to the regenerated catalyst slide valve 2-7 through a pipeline; the light hydrocarbon aromatization slide valve 3-9 is connected to the naphtha-to-aromatics reactor shell 1-1 through a pipeline.

In another preferred embodiment, the product gas delivery pipe II 3-8 is connected to the naphtha-to-aromatics reactor shell 1-1.

In order to achieve aromatization of liner and branched aliphatic hydrocarbons, increase the yield of aromatic hydrocarbons and the content of p-xylene in mixed xylenes, the present application provides a method for preparing aromatic hydrocarbons from naphtha, the method using any of the above-mentioned naphtha-to-aromatics devices.

Furthermore, the method comprises: introducing a raw material and a high-temperature catalyst into the light hydrocarbon aromatization reactor to produce a light hydrocarbon aromatization product gas;
introducing naphtha, the catalyst from the light hydrocarbon aromatization reactor, and the light hydrocarbon aromatization product gas into the naphtha-to-aromatics reactor to generate a product gas flow containing BTX.

In a preferred embodiment, the catalyst is a metal molecular sieve bifunctional catalyst.

The metal molecular sieve bifunctional catalyst adopts metal-modified HZSM-5 zeolite molecular sieve;
the metal used for metal modification is at least one selected from the group consisting of La, Zn, Ga, Fe, Mo, and Cr;
the metal modification method comprises: placing the HZSM-5 zeolite molecular sieve in a metal salt solution, impregnating, drying and calcining to obtain the metal modified HZSM-5 zeolite molecular sieve.

In a preferred embodiment, the method comprises the following steps:
a) naphtha enters the naphtha-to-aromatics reaction zone of the naphtha-to-aromatics reactor 1 through the naphtha-to-aromatics reactor distributor 1-2, contacts with the catalyst from the light hydrocarbon aromatization reactor 3, generates a product gas flow containing BTX, light olefins, hydrogen, light alkanes, combustible gas, heavy aromatics and unconverted naphtha, and at the same time, the catalyst is coked and converted into the spent catalyst;
   the product gas flow enters the gas-solid separation unit I 1-3 to remove the spent catalyst carried therein, then enters the gas collection chamber I 1-4, and enters the downstream section through the product gas delivery pipe I 1-5; the spent catalyst in the naphtha-to-aromatics reaction zone enters the reactor stripper 1-6 through the open end of the inlet pipe of the reactor stripper 1-6, is stripped, and after stripping, enters the regenerator 2 through the spent catalyst slide valve 1-7 and the spent catalyst delivery pipe 1-8;
b) passing the regenerated gas into the regeneration zone of the regenerator 2 through the regenerator distributor 2-2, and contacting with the spent catalyst, the coke on the spent catalyst reacts with the regenerated gas to generate flue gas, and at the same time, the spent catalyst is converted into the regenerated catalyst; the flue gas enters the regenerator gas-solid separation unit 2-3 to remove the regenerated catalyst carried therein, and then enters the regenerator gas collection chamber 2-4, and enters the downstream section through the flue gas delivery pipe 2-5; the regenerated catalyst passes through the regenerator stripper 2-6, the regenerated catalyst slide valve 2-7 in sequence to enter the light hydrocarbon aromatization reactor 3;
c) feeding the riser reactor feedstock into the riser reactor through the inlet end of the riser reactor 3-1, contacting and reacting with the regenerated catalyst from the regenerator, and converting the riser reactor feedstock into a flow containing components such as BTX, light olefins and H₂ under the action of the catalyst, and then entering the inner lower part of the light hydrocarbon aromatization reaction zone in the bed reactor through the outlet end of the riser reactor 3-3; feeding the bed reactor feedstock into the light hydrocarbon aromatization reaction zone through the bed reactor distributor 3-5, contacting with the catalyst from the riser reactor, and generating a light hydrocarbon aromatization product gas containing components such as BTX, light olefins and H₂; the light hydrocarbon aromatization product gas enters the gas-solid separation unit II 3-6 to remove the catalyst carried therein, and then enters the gas collection chamber II 3-7, and then enters the gas-solid separation zone of the naphtha-to-aromatics reactor through the product gas delivery pipe II 3-8; the catalyst in the light hydrocarbon aromatization reaction zone passes through the light hydrocarbon aromatization slide valve 3-9 to enter the naphtha-to-aromatics reactor 1.

The light olefins are ethylene and propylene.

The light alkanes are ethane and propane.

The combustible gas includes methane, CO and the like.

The heavy aromatic hydrocarbons refer to aromatic hydrocarbons having 9 or more carbon atoms in the molecule.

In a preferred embodiment, the naphtha is selected from at least one of coal direct liquefaction naphtha, coal indirect liquefaction naphtha, straight run naphtha and hydrocracked naphtha.

In a preferred embodiment, the naphtha further comprises unconverted naphtha separated from the product gas flow.

In a preferred embodiment, the carbon content in the spent catalyst is in a range from 1.0 wt% to 3.0 wt%.

In a preferred embodiment, the process conditions for the naphtha-to-aromatics reaction zone are: gas superficial velocity of 0.5-2.0 m/s, reaction temperature of 500-650°C, reaction pressure of 100-500 kPa, bed density of 150-700 kg/m³.

Optionally, the gas superficial velocity is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the reaction temperature is independently selected from any value among 500°C, 520°C, 530°C, 540°C, 550°C, 560°C, 570°C, 580°C, 590°C, 600°C, 610°C, 620°C, 630°C, 640°C, 650°C or any range between two values.

Optionally, the reaction pressure is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among 150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

In a preferred embodiment, the carbon content in the regenerated catalyst is ≤0.5 wt%.

In a preferred embodiment, the regeneration gas is at least one selected from the group consisting of oxygen, air and oxygen-enriched air.

In a preferred embodiment, the process conditions for the regeneration zone of the regenerator are: gas superficial velocity of 0.5-2.0 m/s, regeneration temperature of 600-750°C, regeneration pressure of 100-500 kPa, bed density of 150-700 kg/m³.

Optionally, the gas superficial velocity is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the regeneration temperature is independently selected from any value among 600°C, 615°C, 630°C, 645°C, 670°C, 685°C, 700°C, 715°C, 730°C, 745°C, 750°C or any range between two values.

Optionally, the regeneration pressure is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density is independently selected from any value among150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

In a preferred embodiment, the riser reactor feedstock comprises water vapor and light alkanes separated from the product gas flow.

In a preferred embodiment, the water vapor content in the riser reactor feedstock is in a range from 0 wt% to 80 wt%.

In a preferred embodiment, the process conditions for the riser reactor are: gas superficial velocity of 3.0-10.0 m/s, temperature of 580-700°C, pressure of 100-500 kPa, bed density of 50-150 kg/m³.

Optionally, the gas superficial velocity for the riser reactor is independently selected from any value among 3.0m/s, 3.5m/s, 4.0m/s, 4.5m/s, 5.0m/s, 5.5m/s, 6.0m/s, 6.5m/s, 7.0m/s, 7.5m/s, 8.0m/s, 8.5m/s, 9.0m/s, 9.5m/s, 10.0m/s or any range between two values.

Optionally, the temperature for the riser reactor is independently selected from any value among 580°C, 590°C, 600°C, 610°C, 620°C, 630°C, 640°C, 650°C, 660°C, 670°C, 680°C, 690°C, 700°C or any range between two values.

Optionally, the pressure of the riser reactor is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density for the riser reactor is independently selected from any value among 50kg/m³, 60kg/m³, 70kg/m³, 80kg/m³, 90kg/m³, 100kg/m³, 110kg/m³, 120kg/m³, 130kg/m³, 140kg/m³, 150kg/m³ or any range between two values.

The bed reactor feedstock comprises C₄ and C₅ hydrocarbons.

In a preferred embodiment, the bed reactor feedstock comprises C₃, C₄ and C₅ hydrocarbons.

In a preferred embodiment, the C₃, C₄ and C₅ hydrocarbons are derived from C₃, C₄ and C₅ hydrocarbons separated from the product gas flow.

The C₃, C₄ and C₅ hydrocarbons refer to hydrocarbons with 3, 4 and 5 carbon atoms.

In a preferred embodiment, the process conditions for the light hydrocarbon aromatization reaction zone are: gas superficial velocity of 0.5-2.0 m/s, reaction temperature of 550-665°C, reaction pressure of 100-500 kPa, bed density of 150-700 kg/m³.

Optionally, the gas superficial velocity for the light hydrocarbon aromatization reaction zone is independently selected from any value among 0.5m/s, 0.6m/s, 0.7m/s, 0.8m/s, 0.9m/s, 1.0m/s, 1.1m/s, 1.2m/s, 1.3m/s, 1.4m/s, 1.5m/s, 1.6m/s, 1.7m/s, 1.8m/s, 1.9m/s, 2.0m/s or any range between two values.

Optionally, the reaction temperature for the light hydrocarbon aromatization reaction zone is independently selected from any value among 550°C, 560°C, 570°C, 580°C, 590°C, 600°C, 610°C, 620°C, 630°C, 640°C, 650°C, 660°C, 665°C or any range between two values.

Optionally, the reaction pressure for the light hydrocarbon aromatization reaction zone is independently selected from any value among 100kPa, 125kPa, 150kPa, 175kPa, 200kPa, 225kPa, 250kPa, 275kPa, 300kPa, 325kPa, 350kPa, 375kPa, 400kPa, 425kPa, 450kPa, 475kPa, 500kPa or any range between two values.

Optionally, the bed density for the light hydrocarbon aromatization reaction zone is independently selected from any value among 150kg/m³, 200kg/m³, 250kg/m³, 300kg/m³, 350kg/m³, 400kg/m³, 450kg/m³, 500kg/m³, 550kg/m³, 600kg/m³, 650kg/m³, 700kg/m³ or any range between two values.

In the embodiment described in the present application, the naphtha feedstock has a latent aromatic content of 0-80wt%, the single-pass conversion rate of naphtha is 70-95wt%, the unconverted naphtha is separated from the product gas and returned to the naphtha-to-aromatics reactor as a raw material, some light alkanes are separated from the product gas and returned to the riser reactor in the light hydrocarbon aromatization reactor as a raw material, C₃, C₄ and C₅ hydrocarbons are separated from the product gas and returned to the bed reactor in the light hydrocarbon aromatization reactor as a raw material, and the final product distribution is: 60-75wt% BTX, 6-14wt% light olefins, 3-7wt% hydrogen, 3-8wt% light alkanes, 4-6wt% combustible gas, 4-8wt% heavy aromatics, and 0.5-1wt% coke. The content of p-xylene in the mixed xylene in the product is 50-65 wt%.

### Example 1

This embodiment employs the device illustrated in Fig. 1.

Naphtha feedstock fed to the naphtha-to-aromatics reactor is coal direct liquefaction naphtha with an aromatics potential content of 78 wt%, including unconverted naphtha recycled from the separated product gas flow.

Naphtha-to-aromatics reaction zone conditions: gas superficial velocity is 0.5 m/s, reaction temperature is 645°C, reaction pressure is 100 kPa, bed density is 700 kg/m³.

Regeneration gas is air.

Regeneration zone conditions in the regenerator: gas superficial velocity is 0.5 m/s, regeneration temperature is 745°C, regeneration pressure is 100 kPa, bed density is 700 kg/m³.

Riser reactor feedstock is light alkanes separated from the product gas flow.

Riser reactor conditions: gas superficial velocity is 3.0 m/s, temperature is 690°C, pressure is 100 kPa, bed density is 150 kg/m³.

Bed reactor feedstock is C₃, C₄, and Cs hydrocarbons separated from the product gas flow.

Light hydrocarbon aromatization reaction zone conditions: gas superficial velocity is 0.5 m/s, reaction temperature is 665°C, reaction pressure is 100 kPa, bed density is 700 kg/m³.

Carbon content in the spend catalyst is 1.2wt%, and carbon content in the regenerated catalyst is 0.2 wt%.

Single-pass conversion of naphtha feedstock into the naphtha-to-aromatics reactor is 70 wt%.

Product distribution: 74.4 wt% BTX, 6 wt% light olefins, 3 wt% hydrogen, 4 wt% light alkanes, 5 wt% combustible gas, 7 wt% heavy aromatics, 0.6 wt% coke. The content of p-xylene in the mixed xylene in the product is 51 wt%.

### Example 2

This embodiment employs the device illustrated in Fig. 1.

Naphtha feedstock fed to the naphtha-to-aromatics reactor is coal direct liquefaction naphtha with an aromatics potential content of 0.1 wt%, including unconverted naphtha recycled from the separated product gas flow.

Naphtha-to-aromatics reaction zone conditions: gas superficial velocity is 2.0 m/s, reaction temperature is 510°C, reaction pressure is 500 kPa, bed density is 150 kg/m³.

Regeneration gas is oxygen.

Regeneration zone conditions in the regenerator: gas superficial velocity is 2.0 m/s, regeneration temperature is 610°C, regeneration pressure is 500 kPa, bed density is 150 kg/m³.

Riser reactor feedstock is light alkanes separated from the product gas flow and water vapor, with a water vapor content of 80 wt%.

Riser reactor conditions: gas superficial velocity is 10.0 m/s, temperature is 580°C, pressure is 500 kPa, bed density is 50 kg/m³.

Bed reactor feedstock is C₃, C₄, and Cs hydrocarbons separated from the product gas flow.

Light hydrocarbon aromatization reaction zone conditions: gas superficial velocity is 2.0 m/s, reaction temperature is 550°C, reaction pressure is 500 kPa, bed density is 150 kg/m³.

Carbon content in the spend catalyst is 2.9 wt%, and carbon content in the regenerated catalyst is 0.1 wt%.

Single-pass conversion of naphtha feedstock into the naphtha-to-aromatics reactor is 76 wt%.

Product distribution: 66 wt% BTX, 11.3 wt% light olefins, 6 wt% hydrogen, 3 wt% light alkanes, 5 wt% combustible gas, 8 wt% heavy aromatics, 0.7 wt% coke. The content of p-xylene in the mixed xylene in the product is 62 wt%.

### Example 3

This embodiment employs the device illustrated in Fig. 1.

Naphtha feedstock fed to the naphtha-to-aromatics reactor is coal direct liquefaction naphtha with an aromatics potential content of 3 wt%, including unconverted naphtha recycled from the separated product gas flow.

Naphtha-to-aromatics reaction zone conditions: gas superficial velocity is 1.2 m/s, reaction temperature is 550°C, reaction pressure is 120 kPa, bed density is 260 kg/m³.

Regeneration gas is oxygen-enriched air.

Regeneration zone conditions in the regenerator: gas superficial velocity is 1.2 m/s, regeneration temperature is 650°C, regeneration pressure is 120 kPa, bed density is 260 kg/m³.

Riser reactor feedstock is light alkanes separated from the product gas flow and water vapor, with a water vapor content of 25 wt%.

Riser reactor conditions: gas superficial velocity is 7.0 m/s, temperature is 630°C, pressure is 120 kPa, bed density is 80 kg/m³.

Bed reactor feedstock is C₄ and C₅ hydrocarbons separated from the product gas flow.

Light hydrocarbon aromatization reaction zone conditions: gas superficial velocity is 1.2 m/s, reaction temperature is 580°C, reaction pressure is 120 kPa, bed density is 260 kg/m³.

Carbon content in the spend catalyst is 2.0 wt%, and carbon content in the regenerated catalyst is 0.3 wt%.

Single-pass conversion of naphtha feedstock into the naphtha-to-aromatics reactor is 94 wt%.

Product distribution: 60 wt% BTX, 14 wt% light olefins, 7 wt% hydrogen, 8 wt% light alkanes, 4.5 wt% combustible gas, 6 wt% heavy aromatics, 0.5 wt% coke. The content of p-xylene in the mixed xylene in the product is 65 wt%.

### Example 4

This embodiment employs the device illustrated in Fig. 1.

Naphtha feedstock fed to the naphtha-to-aromatics reactor is coal direct liquefaction naphtha with an aromatics potential content of 46 wt%, including unconverted naphtha recycled from the separated product gas flow.

Naphtha-to-aromatics reaction zone conditions: gas superficial velocity is 1.8 m/s, reaction temperature is 600°C, reaction pressure is 200 kPa, bed density is 220 kg/m³.

Regeneration gas is air.

Regeneration zone conditions in the regenerator: gas superficial velocity is 1.8 m/s, regeneration temperature is 700°C, regeneration pressure is 200 kPa, bed density is 220 kg/m³.

Riser reactor feedstock is light alkanes separated from the product gas flow and water vapor, with a water vapor content of 50 wt%.

Riser reactor conditions: gas superficial velocity is 5.0 m/s, temperature is 660°C, pressure is 220 kPa, bed density is 110 kg/m³.

Bed reactor feedstock is C₄ and C₅ hydrocarbons separated from the product gas flow.

Light hydrocarbon aromatization reaction zone conditions: gas superficial velocity is 1.8 m/s, reaction temperature is 630°C, reaction pressure is 200 kPa, bed density is 220 kg/m³.

Carbon content in the spend catalyst is 1.7 wt%, and carbon content in the regenerated catalyst is 0.1 wt%.

Single-pass conversion of naphtha feedstock into the naphtha-to-aromatics reactor is 87 wt%.

Product distribution: 70 wt% BTX, 10 wt% light olefins, 6 wt% hydrogen, 5 wt% light alkanes, 4 wt% combustible gas, 4 wt% heavy aromatics, 1.0 wt% coke. The content of p-xylene in the mixed xylene in the product is 63 wt%.

### Example 5

This embodiment employs the device illustrated in Fig. 1.

Naphtha feedstock fed to the naphtha-to-aromatics reactor is coal direct liquefaction naphtha with an aromatics potential content of 64 wt%, including unconverted naphtha recycled from the separated product gas flow.

Naphtha-to-aromatics reaction zone conditions: gas superficial velocity is 1.0 m/s, reaction temperature is 580°C, reaction pressure is 150 kPa, bed density is 350 kg/m³.

Regeneration gas is air.

Regeneration zone conditions in the regenerator: gas superficial velocity is 1.0 m/s, regeneration temperature is 680°C, regeneration pressure is 150 kPa, bed density is 350 kg/m³.

Riser reactor feedstock is light alkanes separated from the product gas flow and water vapor, with a water vapor content of 40 wt%.

Riser reactor conditions: gas superficial velocity is 7.0 m/s, temperature is 650°C, pressure is 150 kPa, bed density is 80 kg/m³.

Bed reactor feedstock is C₄ and C₅ hydrocarbons separated from the product gas flow.

Light hydrocarbon aromatization reaction zone conditions: gas superficial velocity is 1.0 m/s, reaction temperature is 610°C, reaction pressure is 150 kPa, bed density is 350 kg/m³.

Carbon content in the spend catalyst is 1.5 wt%, and carbon content in the regenerated catalyst is 0.5 wt%.

Single-pass conversion of naphtha feedstock into the naphtha-to-aromatics reactor is 77 wt%.

Product distribution: 71 wt% BTX, 8 wt% light olefins, 5 wt% hydrogen, 3 wt% light alkanes, 6 wt% combustible gas, 6 wt% heavy aromatics, 1.0 wt% coke. The content of p-xylene in the mixed xylene in the product is 57 wt%.

The above examples are merely few examples of the present application, and do not limit the present application in any form. Although the present application is disclosed as above with preferred examples, the present application is not limited thereto. Some changes or modifications made by any technical personnel familiar with the profession using the technical content disclosed above without departing from the scope of the technical solutions of the present application are equivalent to equivalent implementation cases and fall within the scope of the technical solutions.

## Claims

1. A naphtha-to-aromatics fluidized bed device, **characterized in that** the device comprises: a light hydrocarbon aromatization reactor; and a naphtha-to-aromatics reactor; wherein
the light hydrocarbon aromatization reactor includes at least one inlet for introducing a raw material and a high-temperature catalyst; and at least one outlet of the light hydrocarbon aromatization reactor is connected to the naphtha-to-aromatics reactor for delivering catalyst and generated light hydrocarbon aromatization product gas to the naphtha-to-aromatics reactor;
the naphtha-to-aromatics reactor is configured to introduce naphtha to react with a catalyst from the light hydrocarbon aromatization reactor to produce a product gas flow containing BTX.

2. The naphtha-to-aromatics fluidized bed device according to claim 1, **characterized in that** the naphtha-to-aromatics reactor is further provided with a product gas delivery pipe I for outputting the product gas flow containing BTX to a downstream section.

3. The naphtha-to-aromatics fluidized bed device according to claim 1, **characterized in that** the device further comprises a regenerator, and at least one inlet of the light hydrocarbon aromatization reactor is connected to the regenerator for obtaining a high-temperature regenerated catalyst generated by the regenerator.

4. The naphtha-to-aromatics fluidized bed device according to claim 1, **characterized in that** the light hydrocarbon aromatization reactor is divided from top to bottom into at least a gas-solid separation zone II and a light hydrocarbon aromatization reaction zone which are connected to form a bed reactor; the gas-solid separation zone II is provided with a gas-solid separation unit II and a gas collection chamber II, the gas outlet of the gas-solid separation unit II is connected to the gas collection chamber II, and a bed reactor distributor is provided in the inner lower part of the light hydrocarbon aromatization reaction zone for feeding a bed reactor feedstock.

5. The naphtha-to-aromatics fluidized bed device according to claim 4, **characterized in that** the gas collection chamber II is located at the inner top of the bed reactor.

6. The naphtha-to-aromatics fluidized bed device according to claim 4, **characterized in that** the bed reactor feedstock comprises C₄ and C₅ hydrocarbons.

7. The naphtha-to-aromatics fluidized bed device according to claim 4, **characterized in that** the light hydrocarbon aromatization reactor further comprises a riser reactor in addition to the bed reactor, wherein the inlet end of the riser reactor is connect to the regenerator; the outlet end of the riser reactor extends into the lower section of the light hydrocarbon aromatization reaction zone, with the catalyst outlet of the gas-solid separation unit II located above it.

8. The naphtha-to-aromatics fluidized bed device according to claim 7, **characterized in that** the inlet end of the riser reactor is further configured to introduce a catalyst and a riser reactor feedstock.

9. The naphtha-to-aromatics fluidized bed device according to claim 1, **characterized in that** the naphtha-to-aromatics reactor is divided from top to bottom into at least a gas-solid separation zone I and a naphtha-to-aromatics reaction zone which are connected; the gas-solid separation zone I is provided with a gas-solid separation unit I and a gas collection chamber I; the gas outlet of the gas-solid separation unit I is connect to the gas collection chamber I; the lower section of the naphtha-to-aromatics reaction zone is provided with a naphtha-to-aromatics reactor distributor for feeding naphtha feedstock.

10. The naphtha-to-aromatics fluidized bed device according to claim 9, **characterized in that** the gas-solid separation unit I employs at least one set of gas-solid cyclone separators, each set comprising a first-stage gas-solid cyclone separator and a second-stage gas-solid cyclone separator.

11. The naphtha-to-aromatics fluidized bed device according to claim 9, **characterized in that** the gas collection chamber I is located at the inner top of the naphtha-to-aromatics reactor.

12. The naphtha-to-aromatics fluidized bed device according to claim 9, **characterized in that** the gas collection chamber I is connected to the product gas delivery pipe I.

13. The naphtha-to-aromatics fluidized bed device according to claim 9, **characterized in that** the gas-solid separation zone I is connected to the gas-solid separation zone II, and the naphtha-to-aromatics reaction zone of the naphtha-to-aromatics reactor is connected to the light hydrocarbon aromatization reaction zone.

14. The naphtha-to-aromatics fluidized bed device according to claim 13, **characterized in that** the gas-solid separation zone I is connected to the gas collection chamber II through a product gas delivery pipe II.

15. The naphtha-to-aromatics fluidized bed device according to claim 13, **characterized in that** a light hydrocarbon aromatization slide valve is provided on the pipeline connecting the naphtha-to-aromatics reaction zone of the naphtha-to-aromatics reactor and the light hydrocarbon aromatization reaction zone.

16. The naphtha-to-aromatics fluidized bed device according to claim 13, **characterized in that** the outlet of the light hydrocarbon aromatization reaction zone is positioned higher than the inlet of the naphtha-to-aromatics reaction zone of the naphtha-to-aromatics reactor.

17. The naphtha-to-aromatics fluidized bed device according to claim 3, **characterized in that** the regenerator is divided from top to bottom into at least a gas-solid separation zone III and a catalyst regeneration zone which are connected; the gas-solid separation zone III is provided with a regenerator gas-solid separation unit and a regenerator gas collection chamber; the gas outlet of the regenerator gas-solid separation unit is connected to the regenerator gas collection chamber; the regenerator gas collection chamber is provided with a flue gas delivery pipe; the lower section of the catalyst regeneration zone is provided with a regenerator distributor for introducing regeneration gas.

18. The naphtha-to-aromatics fluidized bed device according to claim 17, **characterized in that** the catalyst regeneration zone is sequentially connected to the light hydrocarbon aromatization reactor through a regenerator stripper and a regenerated catalyst slide valve; the inlet pipe of the regenerator stripper extends into the regenerator shell, located above the regenerator distributor, and the catalyst outlet end of the regenerator gas-solid separation unit is located above the open end of the inlet pipe of the regenerator stripper.

19. The naphtha-to-aromatics fluidized bed device according to claim 17, **characterized in that** the regenerator gas collection chamber is located at the inner top of the regenerator shell.

20. The naphtha-to-aromatics fluidized bed device according to claim 3, **characterized in that** at least one outlet of the naphtha-to-aromatics reactor is further connected to the inlet of the regenerator for delivering a spent catalyst generated in the naphtha-to-aromatics reactor to the regenerator, wherein the regenerator is configured to introduce a regeneration gas and convert the spent catalyst into a regenerated catalyst.

21. The naphtha-to-aromatics fluidized bed device according to claim 20, **characterized in that** the naphtha-to-aromatics reaction zone of the naphtha-to-aromatics reactor is sequentially connected to the inlet of the regenerator through a reactor stripper, a spent catalyst slide valve and a spent catalyst delivery pipe; the inlet pipe of the reactor stripper extends into the naphtha-to-aromatics reactor shell, located above the naphtha-to-aromatics reactor distributor, and the catalyst outlet end of the gas-solid separation unit I is located above the open end of the inlet pipe of the reactor stripper.

22. The naphtha-to-aromatics fluidized bed device according to claim 21, **characterized in that** the catalyst inlet of the regenerator is provided on the regenerator shell.

23. A method for producing aromatics from naphtha, **characterized in that** the method comprises: using the naphtha-to-aromatics fluidized bed device according to any one of claims 1-22 to prepare aromatics.

24. The method for producing aromatics from naphtha according to claim 23, **characterized in that** the method comprises:
introducing the raw material and the high-temperature catalyst into the light hydrocarbon aromatization reactor to generate the light hydrocarbon aromatization product gas;
introducing naphtha and the catalyst and the light hydrocarbon aromatization product gas from the light hydrocarbon aromatization reactor into the naphtha-to-aromatics reactor to generate a product gas flow containing **BTX.**

25. The method for producing aromatics from naphtha according to claim 23 or 24, **characterized in that** the catalyst is a metal molecular sieve bifunctional catalyst.

26. The method for producing aromatics from naphtha according to claim 25, **characterized in that** the metal molecular sieve bifunctional catalyst employs a metal-modified HZSM-5 zeolite molecular sieve;
a metal used for a metal modification is at least one selected from the group consisting of La, Zn, Ga, Fe, Mo, and Cr;
the metal modification comprises: placing an HZSM-5 zeolite molecular sieve in a metal salt solution, and carrying out an impregnation, a drying and a calcination to obtain the metal-modified HZSM-5 zeolite molecular sieve.

27. The method for producing aromatics from naphtha according to claim 24, **characterized in that** the components of the light hydrocarbon aromatization product gas comprise: BTX, light olefins and H₂.

28. The method for producing aromatics from naphtha according to claim 24, **characterized in that** in addition to BTX, the product gas flow containing BTX further comprises: light olefins, hydrogen, light alkanes, combustible gas, heavy aromatics and unconverted naphtha.

29. The method for producing aromatics from naphtha according to claim 27 or 28, **characterized in that** the light olefins refer to ethylene and propylene.

30. The method for producing aromatics from naphtha according to claim 28, **characterized in that** the light alkanes refer to ethane and propane;
the combustible gas comprises methane and CO;
the heavy aromatics refer to aromatic hydrocarbons with nine or more carbon atoms per molecule.

31. The method for producing aromatics from naphtha according to claim 24, **characterized in that** introducing the raw material and the high-temperature catalyst into the light hydrocarbon aromatization reactor to generate light hydrocarbon aromatization product gas specifically comprises:
feeding the bed reactor feedstock into the light hydrocarbon aromatization reaction zone through the bed reactor distributor, contacting with the catalyst introduced into the bed reactor to generate light hydrocarbon aromatization product gas.

32. The method for producing aromatics from naphtha according to claim 31, **characterized in that** the bed reactor feedstock comprises C₄ and C₅ hydrocarbons.

33. The method for producing aromatics from naphtha according to claim 32, **characterized in that** the bed reactor feedstock comprises C₃, C₄ and C₅ hydrocarbons.

34. The method for producing aromatics from naphtha according to claim 32, **characterized in that** the C₄ and C₅ hydrocarbons are derived from C₄ and C₅ hydrocarbons separated from the product gas flow.

35. The method for producing aromatics from naphtha according to claim 33, **characterized in that** the C₃, C₄ and C₅ hydrocarbons are derived from C₃, C₄ and C₅ hydrocarbons separated from the product gas flow.

36. The method for producing aromatics from naphtha according to claim 31, **characterized in that** the process conditions for the light hydrocarbon aromatization reaction zone are: gas superficial velocity of 0.5-2.0 m/s, reaction temperature of 550-665°C, reaction pressure of 100-500 kPa, bed density of 150-700 kg/m³.

37. The method for producing aromatics from naphtha according to claim 31, **characterized in that** the method further comprises: removing the catalyst entrained in the light hydrocarbon aromatization product gas in the gas-solid separation zone II of the light hydrocarbon aromatization reactor.

38. The method for producing aromatics from naphtha according to claim 31, **characterized in that** the catalyst in the light hydrocarbon aromatization reaction zone enters the naphtha-to-aromatics reactor through the light hydrocarbon aromatization slide valve.

39. The method for producing aromatics from naphtha according to claim 37, **characterized in that** removing catalyst entrained in the light hydrocarbon aromatization product gas in the gas-solid separation zone II specifically comprises:
the light hydrocarbon aromatization product gas enters the gas-solid separation unit II to remove the catalyst entrained in it, then enters the gas collection chamber II, and is delivered to the gas-solid separation zone I of the naphtha-to-aromatics reactor through the product gas delivery pipe II.

40. The method for producing aromatics from naphtha according to claim 24, **characterized in that** introducing naphtha into the naphtha-to-aromatics reaction zone of the naphtha-to-aromatics reactor through the naphtha-to-aromatics reactor distributor, contacting with the catalyst from the light hydrocarbon aromatization reactor to generate a product gas flow containing BTX, while the catalyst becomes coked and converts into the spent catalyst.

41. The method for producing aromatics from naphtha according to claim 40, **characterized in that** the method further comprises: removing the spent catalyst entrained in the product gas flow containing BTX in the gas-solid separation zone I of the naphtha-to-aromatics reactor and delivering to downstream section.

42. The method for producing aromatics from naphtha according to claim 24, **characterized in that** the naphtha is at least one selected from the group consisting of coal direct liquefaction naphtha, coal indirect liquefaction naphtha, straight-run naphtha and hydrocracked naphtha.

43. The method for producing aromatics from naphtha according to claim 42, **characterized in that** the naphtha further comprises unconverted naphtha separated from the product gas flow, wherein the main components of the unconverted naphtha are linear aliphatic hydrocarbons, branched aliphatic hydrocarbons and naphthenes of C₄-C₁₂.

44. The method for producing aromatics from naphtha according to claim 40, **characterized in that** the process conditions for the naphtha-to-aromatics reaction zone are: gas superficial velocity of 0.5-2.0 m/s, reaction temperature of 500-650°C, reaction pressure of 100-500 kPa, bed density of 150-700 kg/m³.

45. The method for producing aromatics from naphtha according to claim 24, **characterized in that** the method further comprises: introducing the regeneration gas and the spent catalyst into the regenerator to obtain the high-temperature regenerated catalyst, which is delivered to the light hydrocarbon aromatization reactor.

46. The method for producing aromatics from naphtha according to claim 45, **characterized in that** the regeneration gas is introduced into the regeneration zone of the regenerator through the regenerator distributor.

47. The method for producing aromatics from naphtha according to claim 45, **characterized in that** the regeneration gas is at least one selected from the group consisting of oxygen, air and oxygen-enriched air.

48. The method for producing aromatics from naphtha according to claim 45, **characterized in that** the carbon content in the spent catalyst is in a range from 1.0 wt% to 3.0 wt%.

49. The method for producing aromatics from naphtha according to claim 45, **characterized in that** the carbon content in the regenerated catalyst is ≤0.5 wt%.

50. The method for producing aromatics from naphtha according to claim 45, **characterized in that** the process conditions for the regeneration zone of the regenerator are: gas superficial velocity of 0.5-2.0 m/s, regeneration temperature of 600-750°C, regeneration pressure of 100-500 kPa, bed density of 150-700 kg/m³.

51. The method for producing aromatics from naphtha according to claim 45, **characterized in that** the coke on the spent catalyst reacts with the regeneration gas to generate a flue gas; the flue gas enters the gas-solid separation zone III to remove the regenerated catalyst entrained in it.

52. The method for producing aromatics from naphtha according to claim 51, **characterized in that** the flue gas entering the gas-solid separation zone III to remove the regenerated catalyst entrained in it specifically comprises: the flue gas first enters the regenerator gas-solid separation unit to remove the regenerated catalyst entrained in it, then flows through the regenerator gas collection chamber and flue gas delivery pipe to enter downstream section.

53. The method for producing aromatics from naphtha according to claim 51, **characterized in that** the regenerated catalyst enters the light hydrocarbon aromatization reactor through the regenerator stripper and the regenerated catalyst slide valve.

54. The method for producing aromatics from naphtha according to claim 24, **characterized in that** the method further comprises: feeding the riser reactor feedstock into the inlet end of the riser reactor of the light hydrocarbon aromatization reactor; the regenerated catalyst flows through the regenerator stripper and regenerated catalyst slide valve into the riser reactor, where the riser reactor feedstock is converted into a BTX-containing flow under the action of the regenerated catalyst, which then enters the lower section of the light hydrocarbon aromatization reaction zone in the bed reactor through the outlet end of the riser reactor

55. The method for producing aromatics from naphtha according to claim 54, **characterized in that** the method further comprises: introducing the catalyst into the inlet end of the riser reactor of the light hydrocarbon aromatization reactor, which enters the bed reactor through the riser reactor.

56. The method for producing aromatics from naphtha according to claim 55, **characterized in that** the riser reactor feedstock comprises water vapor and light alkanes separated from the product gas flow.

57. The method for producing aromatics from naphtha according to claim 55, **characterized in that** the water vapor content in the riser reactor feedstock is in a range from 0 wt% to 80 wt%.

58. The method for producing aromatics from naphtha according to claim 55, **characterized in that** the process conditions for the riser reactor are: gas superficial velocity of 3.0-10.0 m/s, temperature of 580-700°C, pressure of 100-500 kPa, bed density of 50-150 kg/m³.

59. The method for producing aromatics from naphtha according to claim 54, **characterized in that** the components of the BTX-containing flow comprise: BTX, light olefins and H₂.

60. The method for producing aromatics from naphtha according to claim 24, **characterized in that** the method further comprises:
the spent catalyst in the naphtha-to-aromatics reaction zone of the naphtha-to-aromatics reactor is introduced into the reactor stripper, stripped, and then flows through the spent catalyst slide valve and the spent catalyst delivery pipe into a downstream section.

61. The method for producing aromatics from naphtha according to claim 60, **characterized in that** the downstream section is the regenerator.
